# EUROPÄISCHE PATENTSCHRIFT

(11) **EP 0 813 399 B1**
(45) Veröffentlichungstag und Bekanntmachung des Hinweises auf die Patenterteilung: **13.09.2006**
(21) Anmeldenummer: 96907379.0
(22) Anmeldetag: 07.03.1996
(51) Int. Cl.: A61F 9/00, A61K 9/19

(54) **DARREICHUNGSFORM ZUR APPLIKATION VON PHARMAZEUTISCHEN WIRKSTOFFEN UND HILFSSTOFFEN UND VERFAHREN ZUR HERSTELLUNG DERSELBEN**
FORM OF ADMINISTRATION FOR APPLYING PHARMACEUTICAL SUBSTANCES AND AUXILIARY SUBSTANCES, AND PROCESS FOR THE PREPARATION THEREOF
FORME GALENIQUE PERMETTANT D'ADMINISTRER DES PRODUITS ET ADDITIFS PHARMACEUTIQUES, ET LEUR PROCEDE DE PRODUCTION

(30) Priorität: 07.03.1995 DE 19508029
(43) Veröffentlichungstag der Anmeldung: 29.12.1997
(73) Patentinhaber: Süverkrüp, Richard, 53332 Bornheim (DE); Grunthal, Sabine, 51061 Köln (DE); Diestelhorst, Michael, Prof. Dr. med., 50933 Köln (DE)
(72) Erfinder: Süverkrüp, Richard, 53332 Bornheim (DE); Grunthal, Sabine, 51061 Köln (DE); Diestelhorst, Michael, Prof. Dr. med., 50933 Köln (DE)
(74) Vertreter: Müller-Gerbes, Margot
(86) Internationale Anmeldenummer: PCT/EP1996/000969
(87) Internationale Veröffentlichungsnummer: WO 1996/027350

(56) Entgegenhaltungen:
- EP-A- 0 064 841
- EP-A- 0 258 683
- WO-A-91/19480
- DE-A- 2 441 191

## Beschreibung

Die Erfindung betrifft eine Darreichungsform zur Applikation von pharmazeutischen Wirkstoffen, pharmazeutischen Hilfsstoffen, Arzneistoffen und/oder Diagnostika, umfassend einen Träger und eine auf dem Träger fixierte Zubereitung, die bei Kontakt mit einer Flüssigkeit während der Applikation von dem Träger ablösbar ist und ein zum Herstellen derselben.

Aus der EP 0064 841 A1 ist eine Darreichungsform für das Inkontaktbringen von pharmazeutischen Agenzien mit Körperfeuchtigkeit bekannt, bei der das pharmazeutische Agens in einer löslichen Matrix, umfassend beispielsweise ein wasserlösliches Polymer, enthalten ist und als Film auf einem Streifen aufgebracht und getrocknet ist.

Der getrocknete das pharmazeutische Agens enthaltende Film ist relativ steif und löst sich bei Kontakt mit Körperfeuchtigkeit nicht immer hinreichend schnell auf. Bei Anwendungen am Aufge kann der getrocknete harte Film auch zu Verletzungen führen.

Die Einbettung von Arzneistoffen in Polymerisate von hydrophilen Monomeren in Gestalt von Polymerlösungen und Emulsionen als Darreichungsform ist seit langem bekannt, wozu beispielhaft auf die DE-PS 26 36 559, CH-PS 625 704 oder für eine Anwendung am Auge DE-OS 24 48 737 verwiesen wird.

Beispielhaft wird auf die EP 0507 224 A2 verwiesen, bei der Augentropfen aus einer ophthalmischen Substanz und einem quellbaren Polysaccharid hergestellt und in das Auge eingetropft werden, wobei sie nach dem Eintropfen aufquellen.

Aus der EP 224 987 A sind Augentropfen bekannt, die eine polymere Komponente in Gestalt von löslichem oder unlöslichem Hyaluronan oder Hylan und ein Arzneimittel enthalten. Die Augentropfen sind in einer wässrigen bis viskoelastischen Lösung vorhanden und werden in das Auge getropft.

Aus der DE 24 41 191 A1 ist eine Darreichungsform bekannt, bei der auf einem Träger eine getrocknete wirkstoffhaltige Einzeldosis aufgebracht ist, wobei als Träger Kunststoff oder Glas, die Flüssigkeiten nicht absorbieren, eingesetzt werden.

Aus der WO 91/19480 ist eine Wirkstoffdosis in der Darreichungsform eines komprimierten gepreßten und lyophilisierten Formteils, wie Tablette, bekannt. Die Komprimierung verbessert zwar die Festigkeit der Darreichungsform, jedoch wird durch die hohe Dichte eine rasche Rehydratisierung verhindert. Des weiteren besteht bei der Herstellung durch das zusätzliche Pressen des Wirkstoffes die Gefahr einer weiteren Kontamination des Produktes durch Mikroorganismen.

Die Art der Applikation von Armzeistoffen wie Ophthalmika in Tropfenform sowie derartige Tropfenlösungen haben eine Reihe Nachteile, wie insbesondere die ungenaue Applikation durch die applizierende Person und die Verträglichkeit der Arzneistofflösungen insbesondere bei Augentropfen sind nicht immer gegeben.

Insbesondere für die Darreichungsform von Ophthalmika stehen folgende Ziele im Vordergrund:
- Vereinfachung von Applikation und Anwendbarkeit
- Verlängerung der Kontaktzeit am Auge
- Steigerung der lokalen Arzneistoffkonzentration
- Verminderung der erforderlichen Dosis
- Reduktion der lokalen und systemischen Nebenwirkungen
- Verminderung des Konservierungsmittelzusatzes mit dem Ziel, Schädigungen von Bindehaut und Tenon bei Langzeitanwendern zu reduzieren.

Für Arzneistoffe, Hilfsstoffe, Diagnostika bleibt stets das Ziel, eine Darreichungsform mit verbesserter Dosiergenauigkeit, chemischer Stabilität des Arzneistoffes durch Abwesenheit von Wasser und Konservierungsmittelfreiheit zu schaffen.

Der Erfindung liegt die Aufgabe zugrunde, eine gegenüber konventionellen Applikationen von pharmazeutischen Wirkstoffen und Hilfsstoffen sowie Diagnostika eine besser verträgliche und besser dosierbare und einfacher handhabbare Darreichungsform zu entwickeln. Des weiteren ist es eine Aufgabe der Erfindung, daß die Lagerstabilität insbesondere hydrolyseempfindlicher Arzneistoffe zu erhöhen. Eine weitere Aufgabe der Erfindung ist darin zu sehen, die Dauer der Freigabe von appliziertem Arzneistoff mittels der Darreichungsform variabel zu gestalten, insbesondere zu verlängern, so daß die Häufigkeit des Applizierens mit Hilfe der erfindungsgemäßen Darreichungsform gegenüber konventionellen Darreichungsformen, beispielsweise von Augentropfen verringert werden kann.

Erfindungsgemäß wird zur Lösung der gestellten Aufgabe eine Darreichungsform zur Applikation von pharmazeutischen Wirkstoffen, pharmazeutischen Hilfsstoffen, Arzneistoffen und/oder Diagnostika gemäß Gattungsbegriff vorgeschlagen, die dadurch gekennzeichnet ist, daß der Träger hydrophob bzw. hydrophobiert ist und die Zubereitung den pharmazeutischen Wirkstoff, pharmazeutischen Hilfsstoff, Arzneistoff und/oder Diagnostikum und ein quellbares hydrophiles oder wasserlösliches hydrophiles Polymer enthält und als Lyophilisat ausgebildet ist, wobei die Zubereitung vom Träger zwecks Applikation durch Hydratation der Zubereitung ablösbar ist.
Vorteilhafte Ausgestaltungen der Erfindung sind den kennzeichnenden Merkmalen der Unteransprüche 2 bis 16 entnehmbar. Zum Herstellen der erfindungsgemäßen Darreichungsform wird ein Verfahren gemäß Anspruch 17 vorgeschlagen.

Das erfindungsgemäß entwickelte Applikationssystem ist konservierungsmittelfrei. Als Darreichungsform ist es vorteilhaft zur äußerlichen Applikation geeignet. Die hydrophilisierte Zubereitung enthält den Arzneistoff, Hilfsstoff oder das Diagnostikum eingebettet in ein quellbares oder wasserlösliches Polymergerüst, welches nach der Benetzung mit Flüssigkeit, insbesondere Körperflüssigkeit, wie Schleim, Blut, Tränenflüssigkeit oder einem geeigneten Medium, ein Gel bildet, das sich selbsttätig vom Träger ablöst und als viskose Stofflösung oder Stoffsuspension mit dem Körper in Wirkverbindung kommt. Zur Applikation wird der Träger am Griffende angefaßt und das flexible Ende, auf welchem das wirkstoffhaltige Lyophilisat aufgebracht ist, mit dem gewünschten Körperteil in Berührung gebracht. Es ist möglich, den Träger mit der lyophilisierten Zubereitung - praktisch in Form von "Trockentropfen" - in vielfältiger Weise zu applizieren, beispielsweise:
- Applikation von Medikation intra-, prae- und postopertativ
- Lokalapplikation an Schleimhäuten
- Therapie bakterieller, viraler Infektionen und Mykosen
- dry eye syndrom - zur Applikation künstlicher Tränenflüssigkeit
- Applikation in vorbestehenden Körperhöhlen
- Möglichkeit als Depoteffekt/Retardierung der applizierten Medikation
- Applikation von Ophthalmika am Auge / Bindehaut.

Die erfindungsgemäße lyophilisierte Darreichungsform zeichnet sich insbesondere durch die bessere Verträglichkeit aus, die durch das Fehlen von Konservierungsmitteln erreicht wird, sowie durch eine präzisere Dosierung, die dadurcherreicht wird, daß jede Applikationseinheit nur eine genaue vorbestimmte Einzeldosis enthält.

Gegenüber konventionellen Methoden der Applikation von Wirkstoffen und Hilfsstoffen und Diagnostika hat die erfindungsgemäße Darreichungsform folgende Vorteile:
- Die Dosierung zum Beispiel von Ophthalmika ist präziser, weil jeweils nur die Wirkstoffmenge für eine Applikation auf einem Träger untergebracht ist, und die Deposition der hydratisierten Dispersion im Bindehautsack durch den Anwender besser kontrollierbar ist als das Tropfen und Zählen der Tropfen aus Augentropfenfläschen.
- Konservierungsmittel sind nicht erforderlich, weil die Träger einzeln steril verpackt sind, und die Packung erst unmittelbar vor der Anwendung geöffnet wird. Damit entfallen mögliche nachteilige Wirkungen von Konservierungsmitteln, zum Beispiel die Schädigung von Hornhaut, Bindehaut und Tenon, die bei Konservierungsmittelzusatz in Ophthalmika unvermeidbar ist.
- Weil das Lyophilisat während der Lagerung wasserfrei ist, verbessert sich die Stabilität vor allem hydrolyseempfindlicher Wirkstoffe.
- Gleichzeitig entfällt die Notwendigkeit, die Arzneistofflösungen auf einen für die Stabilität optimalen pH-Wert einzustellen, der in der Regel nicht physiologisch optimal ist. Damit wird die Verträglichkeit der Gabe von Arzneistoffen und anderen Stoffen mittels der erfindungsgemäßen Darreichungsform weiter verbessert.
- Die Dauer der Freigabe kann verlängert werden, so daß die Häufigkeit der Anwendung gegenüber konventionellen Tropfen u.U. verringert werden kann.
- Durch die Nachgiebigkeit bzw. Flexibilität des Trägers wird die Gefahr von Verletzungen bei Berührung der Darreichungsform mit Körperteilen, wie Schleimhäuten oder der Bindehaut oder Hornhaut verringert, die durch Applikatioren herkömmlicher Methoden, wie zum BeispielPipettenspitzen, die aus festerem Material bestehen, auftreten können. Das ist vor allem bei älteren Patienten mit gestörter Feinmotorik von Bedeutung, die die Medikation selbst anwenden.
- Erfindungsgemäß kann durch Auswahl des hydrophilen Polymers die Wirkstofffreigabe innerhalb gewisser Grenzen gesteuert werden.

Die Erfindung ist in der Kombination von drei Elementen, nämlich das Aufbringen eines Tropfens der Lösung eines hydrophilen Polymers, die pharmakologisch wirksame Stoffe enthalten kann, auf einen hydrophoben Träger, und die Herstellung eines wasserfreien Lyophilisats in situ zu sehen.

Nur wenn diese drei Teilaspekte gleichzeitig erfüllt sind, werden die erfindungsgemäßen Ziele und Vorteile verwirklicht.

Dabei stehen folgende Eigenschaften im Vordergrund
1. Die rasche Rehydratisierung beim Kontakt mit einer Flüssigkeit, insbesondere Körperflüssigkeit, wie Tränenflüssigkeit, die dadurch erreicht wird, daß das hydrophile Polymer, in dem ein oder mehrere pharmakologisch wirksame Stoffe gelöst sein können, in Form eines hochporösen Lyophilisats vorliegt.
2. Die Ablösung von dem Träger bei der Rehydratisierung und die Adhäsion an einem Körperteil, zum Beispiel an der Bindehaut, die durch die Kombination des hydrophilen Polymers mit dem hydrophoben Träger erreicht wird.
3. Die Abwesenheit von Konservierungsmitteln und die Stabilität hydrolyseempfindlicher Arzneistoffe, deren gemeinsame Voraussetzung die Wasserfreiheit ist.
4. Die Verringerung der Verletzungsgefahr beim Kontakt mit diesem weichen flexiblem Träger.
5. Die präzise Dosierung die Möglichkeit der Dosisverringerung im Vergleich zur Gabe von flüssigen Tropfen aus Einzel- und Mehrdosisbehältnissen.

Gemäß der Erfindung ist die Darreichungsform zur äußeren Applikation von pharmazeutischen und sonstigen Stoffen so ausgebildet, daß ein fester kompakter, jedoch flexibler gegebenenfalls weicher Träger für die Zubereitung vorhanden ist, und der Stoff in wasserfreier Form gelöst oder dispergiert in geeigneten Hilfsstoffen, bevorzugt hydrophilen Polymeren, als Lyophilisat, Copräzipitat oder feste Lösung (auch nachfolgend als Dispersion bezeichnet) auf der Oberfläche des flexiblen hydrophoben oder hydrophob ausgerüsteten Trägers aufgebracht ist. Der hydrophobe oder hydrophob ausgerüstete Träger kann eine Folie, insbesondere Kunststoffolie, ein Vlies, ein textiles Material sein, d.h. ein Flächengebilde, und weist zum Beispiel eine streifenförmige Gestalt auf.

Bei der erfindungsgemäßen Darreichungsform wird das Lyophilisat, nämlich die auf dem Träger aufgebrachte Zubereitung, beim Kontakt mit Wasser hydratisiert. Die Hydratation kann bei der Applikation durch Kontakt mit einer Körperflüssigkeit erfolgen. Die Zubereitung, d.h. das Lyophilisat kann aber auch vor der Anwendung benetzt werden, zum Beispiel durch Eintauchen der Spitze des Trägers in Wasser oder eine wässrige Lösung, um dann die Zubereitung zusammen mit der Flüssigkeit mittels des Trägers zu applizieren. Das Lösungsmittel bzw. Wasser kann jedoch auch aus einem Depot freigesetzt werden, das auf dem Träger angebracht oder darin eingebettet ist, siehe beispielsweise die Ausführungsform gemäß den kennzeichnenden Merkmalen des Anspruches 16.

Die Ablösung der vollständig oder zunächst nur oberflächlich hydratisierten Wirkstoffdispersion vom Träger und ihre Adhäsion am Körper wird erreicht durch zweckmäßige Einstellung der folgenden Einflußgrößen:
- Benetzungsverhalten der trockenen Dispersion,
- Penetration von wäßrigen Lösungen in die Dispersion, deren Quellungs- und Auflösungsverhalten
- ausreichende Adhäsion der hydratisierten Dispersion an dem Körperteil, wie Bindehaut bei geringer Adhäsion am Träger
- Viskosität und Oberflächenspannung der hydratisierten Dispersion.

Als Träger wird ein textiles Flächengebilde aus natürlichen Fasern und/oder synthetischen Fasern und/oder Filamenten bzw. eine Kunststoffolie vorgeschlagen. Es ist jedoch auch möglich, den Träger aus einer Kombination von mehreren Schichten Fasern und Folie herzustellen. Der Träger soll so flexibel sein und weich sein, daß er bei Berührung mit dem Körperteil diesen nicht verletzen kann. Der Träger kann auf Basis eines hydrophoben thermoplastischen Kunststoffes gemäß Anspruch 4 gefertigt oder beschichtet sein, sofern diese physiologisch indifferent sind. Als vliese kommen insbesondere auch Meltblown-Vliese in Frage aus Microfasern, beispielsweise auf Basis Polypropylen, die in sehr geringen Flächengewichten und weicher Ausführung mit hydrophoben Eigenschaften herstellbar sind. Bevorzugt ist der Träger für die Darreichungsform als kleiner Streifen ausgebildet, wobei er einen sehr weichen zungenförmigen Bereich aufweist, auf dem die Zubereitung, enthaltend den Wirkstoff, wie Arzneistoff und das quellbare oder wasserlösliche hydrophile Polymer aufgebracht ist und einen weiteren, insbesondere mittels Verstärkungsschichten etwas steifer ausgebildeten Bereich, der zum Handhaben, d.h. zum Anfassen und Handeln dient. Die Darreichungsform kann einen Träger in einer Breite von etwa 4 bis 8 mm bei einer Länge von etwa 30 bis 50 mm aufweisen.

Es ist wichtig, daß der Träger, auf dem die Zubereitung aufgebracht ist, und zwar als Lyophilisat, eine hydrophobe Oberfläche aufweist. Dies ist nämlich von Bedeutung, wenn die Zubereitung zwecks Applikation mit Flüssigkeit in Berührung kommt, wobei die Zubereitung aufquillt, daß die aufquellende oder sich auflösenden Zubereitung infolge der Hydrophobie des Trägers sich leicht von diesem ablöst, d.h. praktisch selbsttätig ablöst und als viskose Arzneistofflösung oder Suspension mucoadhäsiv am Auge oder anderen Körperteilen, Geweben oder dergleichen haftet und dort zur Wirkung kommt.

Eine Reihe von Trägermaterialien, wie beispielsweise fluorhaltige Polymere, weisen ausreichend große Hydrophobie auf, so daß sie sich besonders gut als Trägermaterial für die erfindungsgemäße Darreichungsform eignen. Andererseits ist es auch möglich, textile Gewebe aus Natur und/oder Kunststoffasern mittels aus der Textilindustrie bekannter Hydrophobiertechniken und Hydrophobiermittel entsprechend hydrophob auszurüsten.

Die erfindungsgemäß als Xerogel-Zubereitung ausgebildete Arzneimittelgabe ist lyophilisiert, d.h. gefriergetrocknet. Hierbei ist der jeweilige Wirkstoff, Hilfsstoff oder dergleichen in wasserfreier Form gelöst oder dispergiert in physiologisch verträglichen hydrophilen Polymeren. Die Zubereitung wird auf dem Träger bevorzugt in kompakter Form als Häufchen oder in Tropfenform aufgebracht. Die Masse des applizierten Xerogels, d.h. der Zubereitung, enthaltend den Wirkstoff, Hilfsstoff oder dergleichen und hydrophiles Polymer, kann jeder Medikation angepaßt werden, von wenig bis zu Grammenge. Bei Ophthalmika beispielsweise kann sie zwischen 0,05 und 0,2 mg betragen, die durch ca. 2 bis 6 Mikroliter Flüssigkeit, beispielsweise Tränenflüssigkeit hydratisiert werden können. Hierbei stellt sich das Problem der Isotonisierung nicht so ausgeprägt, wie bei der Applikation größerer Volumina wässriger Lösungen, wie es bei flüssigen Augentropfen üblich ist, da der osmotische Druck der hydratisierten Oberfläche des Polymergels der erfindungsgemäßen Zubereitung niedriger ist und die Freisetzung des Arzneistoffes nur langsam erfolgt, weil die entstehende Gelschicht als Diffusionsbarriere wirkt.

Der Gewichtsanteil an Arzneistoff in der Zubereitung ist stark abhängig von dem jeweiligen Arzneistoff.

Für die erfindungsgemäße Darreichungsform können im Grundsatz alle Ophthalmika, die äußerlich angewendet werden, am Auge verwendet werden. Insbesondere ist es möglich, sie in der erfindungsgemäßen Weise als lyophilisierte Zubereitung in Verbindung mit einem hydrophilen quellbaren oder wasserlöslichen Polymeren zu applizieren.

Als ophthalmische Arzneistoffe, die in der erfindungsgemäßen Darreichungsform eingesetzt werden können, sind beispielsweise die folgenden Arzneistoffklassen geeignet: Antiallergika, Antibiotika, Chemotherapeutika, Corticosteroide, Antiglaukomatosa, Lokalanästhetika, Mydriatika, nichtsteroidale Antiphlogistika, Alpha-Sympatomimetika, Virustatika.

Grundsätzlich gilt für alle einsetzbaren Arzneistoffe: je geringer der Arzneistoffgehalt einer Einzeldosis ist, um so besser läßt sich der Arzneistoff in die erfindungsgemäße Darreichungsform als Lyophilisat inkorporieren. Der Wirkstoffgehalt der Lyophilisat-Zubereitung kann in einer Größenordnung von bis zu 20 bis 30 Gew.-% des in der lyophilisierten Zubereitung enthaltenden hydrophilen Polymeren liegen.

Bei der Erfindung handelt es sich um eine Darreichungsform für bekannte handelsübliche und zulassungspflichtige pharmazeutische Wirkstoffe, Hilfsstoffe und Diagnostika.

Als hydrophile Polymere und Biopolymere, die entweder quellbar oder wasserlöslich sind, werden insbesondere solche Stoffe, die als physiologisch verträgliche nicht toxische Verdickungsmittel bekannt sind, insbesondere Hydrokolloide eingesetzt, die in wässrigen Systemen Gele bzw. viskose Lösungen bilden.

Hierfür kommen beispielsweise organische natürliche Verdickungsmittel aus der Gruppe der Polysaccharide und Biopolymeren, wie Agar-Agar, Carrageen, Tragant, Gummi arabicum, Alginate, Pektine, Polyosen, Guar-Mehl, Johannisbrotbaumkernmehl, Stärke, Dextrine, Gelatine, Casein, Xanthan, Alginsäure in Frage (siehe Anspruch 16). Aber auch organische abgewandelte Naturstoffe, die als Verdickungsmittel bekannt sind, wie Carboxymethylcellulose, Celluloseether, Hydroxyethyl- und -propylcellulose, Kernmehlether, sind einsetzbar. Aber auch organische vollsynthetische Stoffe als Verdickungsmittel, wie Polyvinylalkohol, Polyvinylpyrrolidon, Poly(meth)acrylsäuren, Poly(meth)acrylate sind geeignet, in der vorliegenden Erfindung eingesetzt zu werden, um ein polymeres quellbares bzw. wasserlösliches Gerüst zu bauen, in das der Arzneistoff eingebettet ist. Polyacrylsäuren und Polyvinylpyrrolidone sind wasserlöslich, während die meisten der anderen aufgeführten Verdickungsmittel überwiegend quellbar sind und Gele bilden. Wesentlich ist, daß der Arzneistoff in quellbares oder wasserlösliches Polymergerüst von hydrophilen Polymeren eingebettet ist, und das Polymergerüst in der Lage ist, bei Kontakt mit einer Flüssigkeit, beispielsweise Tränenflüssigkeit, ein sich von dem Träger ablösendes Gel oder Dispersion zu bilden. Diese Ablösung wird durch die hydrophobe Ausstattung des Trägermaterials gefördert.

Bevorzugt wird die Darreichungsform gemäß der Erfindung so ausgebildet, daß jeweils ein Täger den Wirkstoff, Hilfsstoff, Diagnostikum, Arzneistoff in einer für eine Applikation ausreichenden Menge enthält. Der Träger kann kleiner handhabbarer Streifen ausgebildet sein, auf dem die Zubereitung punktförmig als Häufchen aufsitzt und bei Berührung mit dem Körperteil, wie Auge, Kontakt der Körperflüssigkeit, wie der Tränenflüssigkeit, das Wasser aufnimmt und ein Gel bildet oder eine Dispersion bildet, die sich von dem Träger löst und am Körper, Auge anhaftet. Jeder Träger mit Zubereitung ist steril verpackt, so daß eine einwandfreie Handhabung und Lagerung der erfindungsgemäßen Darreichungsform ermöglicht ist.

Ein Verfahren zum Herstellen einer erfindungsgemäßen Darreichungsform zeichnet sich dadurch aus, daß
a) eine Lösung aus einem quellbaren oder wasserlöslichen, hydrophilen Polymer hergestellt wird,
b) und der pharmazeutische Wirkstoff, pharmazeutische Hilfsstoff, Arzneistoff und/oder das Diagnostikum in diese Lösung eingebracht und darin gelöst oder dispergiert wird, wodurch eine gelöste Zubereitung erhalten wird,
c) die gelöste Zubereitung auf ein Trägermaterial in einer gewünschten Dosis aufgebracht, insbesondere als Tropfen aufgetropft wird,
d) dann das Trägermaterial mit der gelösten aufgebrachten Zubereitung eingefroren und anschließend gefriergetrocknet wird, wordurch die gelöste Zubereitung als Lyophilisat auf dem Trägermaterial erhalten wird,
e) und aus dem die Lyophilisate enthaltenden Trägermaterial die Darreichungsformen mit loyphilisierter Zubereitung konfektioniert werden.

Während des gesamten Herstellungsvorganges werden aseptische Bedingungen gewährleistet sowie die zur Herstellung steriler Produkte erforderlichen an sich bekannten Maßnahmen durchgeführt. Dies gilt sowohl für den Träger als auch die Zubereitung.

Ein besonders vorteilhaftes Verfahren zum Herstellen der Lyophilisat-Zubereitung, enthaltend ein hydrophiles quellbares oder wasserlösliches Polymer und das Arzneimittel oder dergleichen, ist die Gefriertrocknung bzw. Lyophilisation. Die Methoden der Lyophilisierung sind bekannt und hierzu auch handelsübliche Geräte erhältlich. Das charakteristische Merkmal der Gefriertrocknung ist, daß die Entfernung des Lösungsmittels aus einem gefrorenen Präparat durch Sublimation erfolgt. Der sonst bei einer Trocknung stattfindende Phasenübergang flüssig zu gasförmig wird durch die Phasenübergänge flüssig zu fest (einfrieren) und fest zu gasförmig (Sublimation oder Gefriertrocknung im engeren Sinne) ersetzt. Neben zahlreichen anderen physikochemischen Besonderheiten hat dies zur Folge, daß in einem gefriergetrockneten Präparat - Lyophilisat - Polymere oder thermolabile Arzneistoffe (z.B. Proteine) nicht zerstört werden, daß es nicht geschrumpft ist, daß es eine sehr große innere Oberfläche besitzt, daß es imstande ist, sich sekundenschnell zum Beispiel wieder in Wasser aufzulösen oder mit Wasser vollzusaugen. Zusammengefaßt ausgedrückt: Die ursprünglichen Eigenschaften eines Präparates bleiben nach der Gefriertrocknung in optimaler Weise erhalten. Gefriergetrocknete wasserfreie Präparate können bei geeigneter Verpackung sehr lange gelagert werden.

Es ist auch möglich, die erfindungsgemäße Darreichungsform dergestalt weiter zu entwickeln, daß sie ohne Arnzeistoffzugabe hergestellt wird und nur quellbares oder wasserlösliches hydrophiles Polymer als Lyophilisat enthält. Eine derartig ausgebildete Darreichungsform in Verbindung mit einem Flüssigkeitsdepot auf dem Träger gemäß Anspruch 16 kann erfindungsgemäß als Applikator für künstliche Tränenflüssigkeit verwandt werden.

Die Erfindung wird in der Zeichnung an zwei Ausführungsbeispielen erläutert. Es zeigen
- Fig. 1: eine Darreichungsform in Seitenansicht und
- Fig. 2: die Darreichungsform in Draufsicht mit einer Zubereitung
- Fig. 3, 4: eine Darreichungsform in Seitenansicht und Draufsicht mit Zubereitung und Flüssigkeitsdepot.

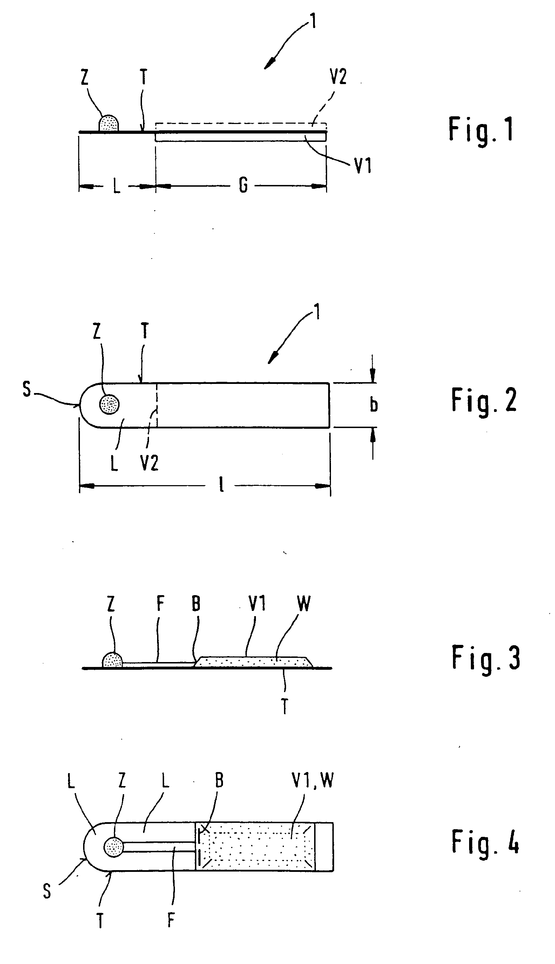

Die Darreichungsform 1 nach Fig. 1 umfaßt den Träger T in Streifenform aus einem flexiblen thermoplastischen Kunststoff, zum Beispiel einer Polytetrafluorethylenfolie einer Dicke von 100 µm, die sehr weich und flexibel ist. Um dem Träger T im Griffbereich G eine höhere Stabilität und Griffigkeit zu verleihen, ist vorgesehen, den Griffbereich mit einer weiteren Schicht, beispielsweise textilem Flächengebilde oder Folie, zu verstärken, so daß zwar die Flexibilität und Bewegbarkeit der Darreichungsform in ihrer Gänze erhalten bleibt, jedoch der nichtverstärkte Bereich als Zunge L weich und anschmiegsam ausgebildet ist, so daß er bei Berührung mit dem Auge und der Hornhaut keine Verletzungen hervorruft. Die Zunge ist in ihrem vorderen Bereich S abgerundet. Insgesamt ist der Träger streifenförmig ausgebildet, wobei er eine Breite b von beispielsweise 0,5 cm bei einer Länge von 3 cm aufweist. Der Griffbereich G sollte hierbei mindestens 2 cm lang sein, so daß ein gutes Handhaben möglich ist. Die Verstärkungsschichten V1, V2 können sowohl auf der Unter- als auch auf Oberseite des Trägers vorhanden sein. Der vordere nicht verstärkte Teil des Trägers C ist dann gegenüber dem verstärkten Bereich des Trägers flexibler. Auf dem weichen, nichtverstärkten, flexiblen, zungenförmigen Teil des Trägers T ist die Zubereitung Z als Lyophilisat, enthaltend zum Beispiel den Arzneistoff und das hydrophile Polymer in kompakter Form, beispielsweise als Häufchen oder in Tropfenform, haftfest bzw. anhaftend aufgebracht. Die in der Fig. 1 und 2 dargestellte Darreichungsform, nämlich ein Arzneimittelstreifen, kann nun im Griffbereich angefaßt werden und die Zubereitung Z in einfacher Weise am Körperteil, zum Beispiel Augenbereich durch Kontakt appliziert werden. Durch die Tränenflüssigkeit des Auges wird die lyophilisierte Zubereitung Z aufgequollen bzw. aufgelöst und löst sich automatisch von dem Träger ab und haftet an der Bindehaut, wo diese viskose Arzneistofflösung oder Suspension mucoadhäsiv zur Wirkung kommt.

Um in jedem Fall sicherzustellen, daß bei der Applikation der lyophilisierten Zubereitung mit der Darreichungsform des Streifens ausreichend Flüssigkeit für die Hydratation der Zubereitung vorhanden ist, kann auch, wie in der Ausführung gemäß Fig. 3 und 4 dargestellt, der Träger, d.h. die Darreichungsform, mit einem Flüssigkeitsreservoir oder -depot, zum Beispiel Wasser für Injektionszwecke oder isotonische Kochsalzlösung, ausgestattet sein. Hierbei ist eine Verstärkungsfolie unter Ausbildung eines Hohlraumes auf dem Träger T befestigt und der Hohlraum mit Flüssigkeit gefüllt. An einer dem zungenförmigen Teil des Trägers mit der Zubereitung Z zugewandten Seite kann die Verstärkung VI, die die Flüssigkeit abdeckt mit einer Sollbruchstelle versehen sein, beispielsweise einem kleinen abreißbaren Blättchen B, so daß im Applikationsfalle dieses Blättchen B entfernt wird und dann die Flüssigkeit aus dem Depot herausfließt. Um diese Flüssigkeit unmittelbar mit der lyophilisierten Zubereitung Z auf dem Träger T in Verbindung zu bringen, ist beispielsweise ein Filterstreifen F auf dem Träger aufgebracht, der von der Zubereitung Z bis zu dem Flüssigkeitsdepot W reicht. Bei Auslaufen der Flüssigkeit aus der geöffneten Sollbruchstelle B wird dieses durch das Filter aufgesaugt und zwangsläufig zur Zubereitung Z geführt, so daß hier wiederum die Zubereitung aufquellen bzw. in Lösung geht und eine Arzneimittellösung oder Suspension entsteht, die sich von dem hydrophoben Träger ablöst und mit der Bindehaut in Berührung kommt.

Als Trägermaterialien kommen alle solche Flächengebilde kompakt, gewebt, gewirkt oder_non-wovens in Frage, die physiologisch unbedenklich sind, die keine Fasern oder Partikel abgeben und die so weich sind, daß sie bei Berührung mit dem Körperteil, wie der Hornhaut bzw. Bindehaut des Auges, keine Verletzungen hervorrufen.

Die Erfindung wurde nur beispielhaft in der Anwendung von Ophthalmika für das Auge dargestellt. Die erfindungsgemäße Darreichungsform kann jedoch in vielfältiger Weise eingesetzt werden, zum Beispiel mit Lyophilisaten, enthaltend pharmazeutische Wirkstoffe, Hilfsstoffe oder Diagnostika oral oder bei Operationen an Wundflächen.

Die erfindungsgemäße Darreichungsform ist nicht auf die dargestellten und beschriebenen Ausführungsbeispiele beschränkt.

## Patentansprüche

1. Darreichungsform zur Applikation von pharmazeutischen Wirkstoffen, pharmazeutischen Hilfsstoffen, Arzneistoffen und/oder Diagnostika, umfassend einen Träger und eine auf dem Träger fixierte Zubereitung, die bei Kontakt mit einer Flüssigkeit während der Applikation von dem Träger ablösbar ist, **dadurch gekennzeichnet, daß** der Träger hydrophob bzw. hydrophobiert ist und die Zubereitung den pharmazeutischen Wirkstoff, pharmazeutischen Hilfsstoff, Arzneistoff und/oder Diagnostikum und ein quellbares hydrophiles oder wasserlösliches hydrophiles Polymer enthält und als Lyophilisat ausgebildet ist, wobei die Zubereitung vom Träger zwecks Applikation durch Hydratation der Zubereitung ablösbar ist.

2. Darreichungsform nach Anspruch 1,
**dadurch gekennzeichnet, daß** der Träger flexibel ist.

3. Darreichungsform nach Anspruch 1 oder 2,
**dadurch gekennzeichnet, daß** der Träger aus einem textilen Flächengebilde aus natürlichen Fasern und/oder synthetischen Fasern und/oder Filamenten und/oder einer Kunststoffolie gebildet ist.

4. Darreichungsform nach einem der Ansprüche 1 bis 3,
**dadurch gekennzeichnet, daß** der Träger auf Basis eines hydrophoben thermoplastischen Kunststoffes, wie Polyolefinen, Ethylencopolymeren, fluorhaltigen Polymeren, wie Polytetrafluorethylen, Polyfluorethylenpropylen, Ethylentetrafluorethylencopolymere, Polyvinylidenfluorid, Perfluoralkoxy, Ethylen-Chloritrifluorethylen-Copolymer, Polychlortrifluorethylen, Polyvinylfluorid, Polytetrafluorethylen-Copolymer mit einem fluorierten zyklischen Ether oder Tetrafluorethylen-Hexafluorpropylen-Vinylidenfluorid-Copolymer gefertigt ist oder der Träüger mit einer Beschichtung aus einem hydrophoben Kunststoff versehen ist.

5. Darreichungsform nach einem der Ansprüche 1 bis 4,
**dadurch gekennzeichnet, daß** die Zubereitung in einer konzentrierten Masse in kompakter Form auf einem Teilbereich des Trägers aufgebracht ist.

6. Darreichungsform nach einem der Ansprüche 1 bis 5,
**dadurch gekennzeichnet, daß** der Träger als Streifen und zumindest ein Ende des Streifens abgerundet ausgebildet ist und innerhalb des abgerundeten Bereiches und/oder im angrenzenden Bereich die Zubereitung auf dem Träger aufgebracht ist. ' .

7. Darreichungsform nach einem der Ansprüche 1 bis 6,
**dadurch gekennzeichnet, daß** der Träger in einem außerhalb des die Zubereitung aufweisenden Bereiches liegenden Bereich zumindest-teilweise ein- und/oder beidseitig verstärkt ausgebildet ist, wobei als Verstärkung haftfest mit dem Träger verbundene Flächengebilde aus Papier, textilen Materialien und/oder thermoplastischem Kunststoff vorgesehen sind.

8. Darreichungsform nach einem der Ansprüche 1 bis 7,
**dadurch gekennzeichnet, daß** der pharmazeutische Wirkstoff, der pharmazeutische Hilfsstoff, Arzneistoff und/oder Diagnostikum in wasserfreier Form in einem hydrophilen Polymer gelöst oder in einem hydrophilen Polymeren dispergiert als hydrophilisierte Zubereitung auf dem Träger vorhanden ist.

9. Darreichungsform nach einem der Ansprüche 1 bis 8,
**dadurch gekennzeichnet, daß** für die Zubereitung als hydrophile physiologisch verträgliche nichttoxische Polymere Verdickungsmittel, insbesondere Hydrokolloide, eingesetzt sind, die in wässrigen Systemen Gele bzw. viskose Lösungen bilden.

10. Darreichungsform nach einem der Ansprüche 1 bis 8,
**dadurch gekennzeichnet, daß** als hydrophile Polymere organische natürliche Verdickungsmittel aus der Gruppe der Polysaccharide und Biopolymeren, wie Agar-Agar, Carrageen, Tragant, Gummi arabicum, Alginate, Pektine, Polyosen, Guar-Mehl, Johannisbrotbaumkernmehl, Stärke, Dextrine, Gelatine, Casein, Xanthan, Alginsäure, eingesetzt sind.

11. Darreichungsform nach einem der Ansprüche 1 bis 8,
**dadurch gekennzeichnet, daß** als hydrophile Polymere Verdichtungsmittel aus organischen abgewandelten Naturstoffen, wie Carboxymethylcellulose, Celluloseether, Hydroxyethyl- und -propylcellulose, Kernmehlether, eingesetzt sind.

12. Darreichungsform nach einem der Ansprüche 1 bis 8,
**dadurch gekennzeichnet, daß** hydrophile polymere Verdickungsmittel aus organischen vollsynthetischen Stoffen, wie Polyvinylalkohol, Polyvinylpyrrolidon, Poly(meth)acrylsäuren, Poly(meth)acrylate, in der Zubereitung eingesetzt sind.

13. Darreichungsform nach einem der Ansprüche 1 bis 12,
**dadurch gekennzeichnet, daß** der Arzneistoff in ein quellbares oder wasserlösliches Polymergerüst von hydrophilen Polymeren eingebettet ist und das Polymergerüst in der Lage ist, bei Kontakt mit einer Flüssigkeit ein sich von dem Träger ablösendes Gel zu bilden.

14. Darreichungsform nach einem der Ansprüche 1 bis 13,
**dadurch gekennzeichnet, daß** der Träger mit hydrophilisierter Zubereitung lagerstabil und konservierungsmittelfrei und wasserfrei ist.

15. Darreichungsform nach einem der Ansprüche 1 bis 14,
**dadurch gekennzeichnet, daß** auf einem Träger pharmazeutischer Wirkstoff, pharmazeutischer Hilfsstoff, Arzneistoff und/oder Diagnostikum in einer für eine Applikation ausreichenden Menge aufgebracht ist.

16. Darreichungsform nach einem der Ansprüche 1 bis 15,
**dadurch gekennzeichnet, daß** auf dem Träger ein Flüssigkeitsdepot, abgedeckt mit einer Verstärkungsschicht aufgebracht ist, das über eine saugfähige eine Dochtwirkung entfaltendes auf dem Träger aufgebrachtes Verbindungsmaterial mit der Zubereitung in Wirkverbindung bringbar ist.

17. Verfahren zum Herstellen einer Darreichungsform nach einem der Ansprüche 1 bis 16,
**dadurch gekennzeichnet, daß**
a) eine Lösung aus einem quellbaren oder wasserlöslichen, hydrophilen Polymer hergestellt wird,
b) und der pharmazeutische Wirkstoff, pharmazeutische Hilfsstoff, Arzneistoff und/oder das Diagnostikum in diese Lösung eingebracht und darin gelöst oder dispergiert wird, wodurch eine gelöste Zubereitung erhalten wird,
c) die gelöste Zubereitung auf ein Trägermaterial in einer gewünschten Dosis aufgebracht, insbesondere als Tropfen aufgetropft wird,
d) dann das Trägermaterial mit der gelösten aufgebrachten Zubereitung eingefroren und anschließend gefriergetrocknet wird, wordurch die gelöste Zubereitung als Lyophilisat auf dem Trägermaterial erhalten wird,
e) und aus dem die Lyophilisate enthaltenden Trägermaterial die Darreichungsformen mit loyphilisierter Zubereitung konfektioniert werden.

## Claims

1. Presentation form for the application of pharmaceutical agents, pharmaceutical auxiliaries, pharmaceuticals and/or diagnostics, comprising a carrier and a preparation fixed to the carrier, the preparation being detachable from the carrier on contact with a liquid during application, **characterized in that** the carrier is hydrophobic or hydrophobized and the preparation contains the pharmaceutical agent, pharmaceutical auxiliary, pharmaceutical and/or diagnostic and a swellable hydrophilic or water-soluble hydrophilic polymer, the preparation being detachable from the carrier for the purpose of application by means of hydration of the preparation.

2. A presentation form as claimed in claim 1, **characterized in that** the carrier is flexible.

3. A presentation form as claimed in claim 1 or 2, **characterized in that** the carrier is formed from a textile fabric made of natural fibers and/or synthetic fibers and/or filaments and/or plastic film.

4. A presentation form as claimed in one of claims 1 to 3, **characterized in that** the carrier is produced on the basis of a hydrophobic thermoplastic, such as polyolefins, ethylene copolymers, fluorine-containing polymers, such as polytetrafluoroethylene, polyfluoroethylenepropylene, ethylene-tetrafluoroethylene copolymer, polyvinylidene fluoride, perfluoroalkoxy, ethylene-chlorotrifluoroethylene copolymer, polychlorotrifluoroethylene, polyvinyl fluoride, polytetrafluoroethylene copolymer with a fluorinated cyclic ether or tetrafluoroethylene-hexafluoropropylene-vinylidene fluoride copolymer or the carrier is provided with a coating of a hydrophobic plastic.

5. A presentation form as claimed in one of claims 1 to 4, **characterized in that** the preparation is applied to the carrier in a concentrated mass in compact form on one part of the carrier.

6. A presentation form as claimed in one of claims 1 to 5, **characterized in that** the carrier is formed as a strip and at least one end of the strip is designed to be rounded off and the preparation is applied to the carrier within the rounded off area and/or in the adjacent area.

7. A presentation form as claimed in one of claims 1 to 6, **characterized in that** the carrier is designed to be at least partially reinforced on one and/or both sides in an area lying outside the area containing the preparation, the reinforcements provided being fabrics made of paper, textile materials and/or thermoplastic connected adhesively to the carrier.

8. A presentation form as claimed in one of claims 1 to 7, **characterized in that** the pharmaceutical agent, the pharmaceutical auxiliary, pharmaceutical and/or diagnostic is present on the carrier as a hydrophilized preparation in anhydrous form dissolved in a hydrophilic polymer or dispersed in a hydrophilic polymer.

9. A presentation form as claimed in one of claims 1 to 8, **characterized in that** the hydrophilic physiologically tolerable nontoxic polymers employed for the preparation are thickeners, in particular hydrocolloids, which form gels or viscous solutions in aqueous systems.

10. A presentation form as claimed in one of claims 1 to 8, **characterized in that** the hydrophilic polymers employed are organic natural thickeners from the polysaccharides and biopolymers group, such as agar-agar, carrageenan, tragacanth, gum arabic, alginates, pectins, polyoses, guar gum, carob bean flour, starch, dextrins, gelatin, casein, xanthan and alginic acid.

11. A presentation form as claimed in one of claims 1 to 8, **characterized in that** the hydrophilic polymers employed are thickeners made of modified organic natural substances, such as carboxymethylcellulose, cellulose ethers, hydroxyethyl- and -propylcellulose, and carob bean flour ethers.

12. A presentation form as claimed in one of claims 1 to 8, **characterized in that** hydrophilic polymeric thickeners made of organic fully synthetic substances, such as polyvinyl alcohol, polyvinylpyrrolidone, poly(meth)acrylic acids, poly(meth)acrylates, are employed in the preparation.

13. A presentation form as claimed in one of claims 1 to 12, **characterized in that** the pharmaceutical is embedded in a swellable or water-soluble polymer structure of hydrophilic polymers and the polymer structure is able to form a gel which detaches form the carrier on contact with a liquid.

14. A presentation form as claimed in one of claims 1 to 13, **characterized in that** the carrier with hydrophilized preparation is stable on storage and preservative-free and anhydrous.

15. A preparation form as claimed in one of claims 1 to 14, **characterized in that** pharmaceutical agent, pharmaceutical auxiliary, pharmaceutical and/or diagnostic are applied to a carrier in an amount sufficient for one application.

16. A presentation form as claimed in one of claims 1 to 15, **characterized in that** a liquid depot, covered with a reinforcing layer, which can be brought into active combination with the preparation via an absorbent connecting material applied to the carrier displaying a wick action is applied to the carrier.

17. A process for producing a presentation form as claimed in one of claims 1 to 16, **characterized in that**
a) a solution of a swellable or water-soluble, hydrophilic polymer is prepared,
b) and the pharmaceutical agent, pharmaceutical auxiliary, pharmaceutical and/or the diagnostic is incorporated into this solution and dissolved or dispersed therein, whereby a dissolved preparation is obtained,
c) the dissolved preparation is applied to a carrier material in a desired dose, in particular adding it as drops,
d) then the carrier material with the dissolved applied preparation is frozen and subsequently freeze-dried, whereby the dissolved preparation is obtained on the carrier material as a lyophilizate,
e) and the presentation forms with lyophilized preparation from the carrier material comprising the lyophilizates are confected.

## Revendications

1. Forme d'administration pour l'application de principes actifs pharmaceutiques, de substances auxiliaires pharmaceutiques, de produits pharmaceutiques et/ou de produits diagnostiques, comprenant un support et une préparation fixée sur le support, qui est séparable du support au contact avec un liquide pendant l'application, **caractérisée en ce que** le support est hydrophobe ou rendu hydrophobe et **en ce que** la préparation contient le principe actif pharmaceutique, la substance auxiliaire pharmaceutique, le produit pharmaceutique et/ou le produit diagnostique et un polymère hydrophile gonflable ou un polymère hydrophile hydrosoluble et **en ce qu'**elle se présente sous la forme de lyophilisat, la préparation étant séparable du support à des fins d'application par hydratation de la préparation.

2. Forme d'administration selon la revendication 1, **caractérisée en ce que** le support est souple.

3. Forme d'administration selon la revendication 1 ou 2, **caractérisée en ce que** le support est constitué d'un article textile plan en fibres naturelles et/ou en fibres et/ou en filaments synthétiques et/ou en feuille synthétique.

4. Forme d'administration selon l'une quelconque des revendications 1 à 3, **caractérisée en ce que** le support est fabriqué à base d'un matériau synthétique hydrophobe thermoplastique, comme les polyoléfines, les copolymères d'éthylène, les polymères contenant du fluor, tels que le polytétrafluoroéthylène, le polyfluoroéthylènepropylène, les copolymères d'éthylène et de tétrafluoroéthylène, le poly(fluorure de vinylidène), le perfluoroalcoxy, le copolymère d'éthylène et de chlorotrifluoroéthylène, le polychlorotrifluoréthylène, le poly(fluorure de vinyle), un copolymère de polytétrafluoroéthylène avec un éther cyclique fluoré ou un copolymère de tétrafluoroéthylène-hexafluoropropylène-fluorure de vinylidène ou **en ce que** le support est pourvu d'un revêtement en matériau synthétique hydrophobe.

5. Forme d'administration selon l'une quelconque des revendications 1 à 4, **caractérisée en ce que** la préparation est appliquée en masse concentrée sous forme compacte sur une zone partielle du support.

6. Forme d'administration selon l'une quelconque des revendications 1 à 5, **caractérisée en ce que** le support se présente sous la forme de bande et au moins une extrémité de la bande présente une forme arrondie et **en ce que** la préparation est appliquée sur le support, à l'intérieur de la zone arrondie et/ou dans la zone attenante.

7. Forme d'administration selon l'une quelconque des revendications 1 à 6, **caractérisée en ce que**, dans une zone qui se situe en dehors de la zone présentant la préparation, le support présente un renforcement au moins partiellement sur une face et/ou sur les deux faces, en prévoyant comme renforcement des articles plats en papier, en matériaux textiles et/ou en matériau synthétique thermoplastique reliés au support par adhérence.

8. Forme d'administration selon l'une quelconque des revendications 1 à 7, **caractérisée en ce que** le principe actif pharmaceutique, la substance auxiliaire pharmaceutique, le produit pharmaceutique et/ou le produit diagnostique sous forme exempte d'eau sont présents dissous dans un polymère hydrophile ou dispersés dans un polymère hydrophile sous la forme d'une préparation hydrophilisée sur le support.

9. Forme d'administration selon l'une quelconque des revendications 1 à 8, **caractérisée en ce que**, pour la préparation, on utilise comme polymères non toxiques hydrophiles physiologiquement compatibles, des épaississants, en particulier des hydrocolloïdes qui forment des gels ou des solutions visqueuses dans des systèmes aqueux.

10. Forme d'administration selon l'une quelconque des revendications 1 à 8, **caractérisée en ce que** l'on utilise comme polymères hydrophiles des épaississants organiques naturels du groupe des polysaccharides et des biopolymères, comme l'agar-agar, le carragheen, la gomme tragacante, la gomme arabique, les alginates, les pectines, les polyoses, la farine de guar, la farine de graines de caroube, l'amidon, les dextrines, les gélatines, la caséine, le xanthane et l'acide alginique.

11. Forme d'administration selon l'une quelconque des revendications 1 à 8, **caractérisée en ce que** l'on utilise comme polymères hydrophiles des épaississants formés de substances naturelles organiques transformées, comme la carboxyméthylcellulose, l'éther de cellulose, l'hydroxyéthylcellulose et l'hydroxypropylcellulose et l'éther de fleur de farine.

12. Forme d'administration selon l'une quelconque des revendications 1 à 8, **caractérisée en ce que** l'on utilise dans la préparation des épaississants polymères hydrophiles de substances organiques entièrement synthétiques, comme l'alcool polyvinylique, la polyvinylpyrrolidone, les acides poly(méth)acryliques et les poly(méth)acrylates.

13. Forme d'administration selon l'une quelconque des revendications 1 à 12, **caractérisée en ce que** le produit pharmaceutique est noyé dans une ossature polymère de polymères hydrophiles gonflable ou hydrosoluble et **en ce que** l'ossature polymère est en mesure de former un gel se détachant du support au contact d'un liquide.

14. Forme d'administration selon l'une quelconque des revendications 1 à 13, **caractérisée en ce que** le support avec la préparation hydrophilisée est stable au stockage et exempt de conservateur et d'eau.

15. Forme d'administration selon l'une quelconque des revendications 1 à 14, **caractérisée en ce que** l'on applique sur un support un principe actif pharmaceutique, une substance auxiliaire pharmaceutique, un produit pharmaceutique et/ou un produit diagnostique en quantité suffisante pour l'application.

16. Forme d'administration selon l'une quelconque des revendications 1 à 15, **caractérisée en ce qu'**un dépôt liquide, recouvert d'une couche de renforcement, est appliqué sur le support, dépôt qui peut être amené à coopérer avec la préparation via un matériau de liaison absorbant appliqué sur le support et déployant un effet de mèche.

17. Procédé de fabrication d'une forme d'administration selon l'une quelconque des revendications 1 à 16,
**caractérisé en ce que**
a) une solution est fabriquée à partir d'un polymère hydrophile gonflable ou hydrosoluble,
b) et le principe actif pharmaceutique, la substance auxiliaire pharmaceutique, le produit pharmaceutique et/ou le produit diagnostique sont incorporés à cette solution et y sont dissous ou dispersés de manière à obtenir une préparation dissoute,
c) la préparation dissoute est appliquée sur un matériau de support en dose souhaitée, en particulier sous la forme de de gouttes,
d) le matériau de support est ensuite figé avec la préparation dissoute appliquée, puis lyophilisé de manière à obtenir la préparation dissoute sous forme de lyophilisat sur le matériau de support,
e) et les formes d'administration avec la préparation lyophilisée sont élaborées à partir du matériau de support contenant les lyophilisats.
